# FASCICULE DE BREVET EUROPEEN

(11) **EP 0 485 311 B1**
(45) Date de publication et mention de la délivrance du brevet: **12.08.1998**
(21) Numéro de dépôt: 91420391.4
(22) Date de dépôt: 05.11.1991
(51) Int. Cl.: A61F 2/36

(54) **Tige fémorale perfectionnée pour prothèse de hanche**
Oberschenkelteil einer Hüftgelenkprothese
Stem for hip prosthesis

(30) Priorité: 06.11.1990 FR 9014106
(43) Date de publication de la demande: 13.05.1992
(73) Titulaire: Merck Biomaterial France, 26000 Valence (FR)
(72) Inventeur: Collomb, Jean, F-26800 Porte les Valence (FR)
(74) Mandataire: Laurent, Michel

(56) Documents cités:
- EP-A- 0 000 549
- EP-A- 0 241 361
- FR-A- 2 183 230
- FR-A- 2 366 005
- FR-A- 2 640 497
- US-A- 4 608 055
- US-A- 4 963 155

## Description

L'invention concerne une tige fémorale perfectionnée pour prothèse de hanche.

De manière connue, et pour l'essentiel, une tige fémorale pour prothèse de hanche comprend :
- tout d'abord, un col prothétique, destiné à dépasser du fémur et dont l'extrémité présente une tête généralement conique, destinée à recevoir une sphère sur laquelle s'appuie la cupule cotyloïdienne ;
- ensuite, une tige proprement dite, destinée à être insérée dans le fémur à renforcer, comportant une portion proximale raccordée au col prothétique, et une portion distale dont la pointe est engagée dans le canal médullaire du fémur.

Il est bien connu à ce jour de réaliser de telles tiges en une ou deux parties. On a par exemple décrit dans le document FR-A-2 366 005, une tige du type en question, réalisée en deux parties reliées par leur base, et dont la section de raccordement est sensiblement perpendiculaire à l'axe longitudinal de la tige proprement dite. Cette solution qui donne de bons résultats, présente toutefois l'inconvénient de nécessiter de creuser le grand trochanter pour placer l'embase du col prothétique. Or cette opération affaiblit le fémur.

Dans ce type de tige constituée de deux parties, ou même dans les tiges monobloc, lorsque la tige est insérée dans le canal médullaire du fémur, si le chirurgien s'aperçoit que la tête de la tige est mal orientée par rapport au cotyle, il doit modifier le positionnement de la tige dans le canal médullaire, ce qui n'est pas sans poser de problèmes, notamment lorsque la tige est fixée par ciment.

Pour pallier ces inconvénients, on a suggéré dans le document EP-A-0 000 549, de ménager à l'embase du col une couronne dentée qui coopère avec une couronne femelle complémentaire disposée sur le haut de la tige. Cette solution implique également d'entailler le grand trochanter, conduisant une nouvelle fois à l'affaiblissement du fémur.

Pour s'affranchir de ces inconvénients rédhibitoires, on a alors proposé par exemple dans le document FR-A-2 183 230 une tige fémorale en deux parties, dont le col prothétique est rapporté au niveau de la partie supérieure de la tige, et est fixé par la coopération d'une partie mâle issue dudit col prothétique, et d'un orifice complémentaire, ménagé dans la partie supérieure de la tige. Le col est solidarisé au moyen d'un joint fileté, dont l'une des extrémités est fourchue, ladite fourche entourant partiellement la partie mâle issue du col prothétique afin de permettre la solidarisation réversible du col au niveau de la tige. L'avantage d'une telle réalisation réside dans le fait qu'il n'est plus nécessaire de procéder à l'enlèvement de la tige prothétique lors du changement de prothèse, seul le corps prothétique pouvant être changé. Néanmoins, cette réalisation ne permet pas d'orienter à loisir le col prothétique selon un angle d'antéversion donné par rapport au plan général de la tige, compte tenu du système même de solidarisation du col sur la tige.

Par ailleurs, afin de s'affranchir de l'inconvénient inhérent à la fragilisation du grand torchanter, lors de la mise en place de la tige au sein du canal médullaire, on a alors proposé, par exemple dans le document US-A-4 963 155, de prévoir une embase de raccordement non perpendiculaire à l'axe longitudinal de la tige. Cependant, la tige fémorale de la prothèse décrite dans ce document comporte également une superstructure prolongeant l'extrémité supérieure de la tige, allongeant le bras de levier constitué par le col, et augmentant de manière rédhibitoire les risques de luxation de l'articulutation de la hanche.

L'invention pallie ces différents inconvénients. Elle propose une tige du type en question, réalisée en deux partie distinctes, mais dans laquelle il n'est plus nécessaire d'entailler le grand trochanter pour la mise en place et l'orientation adéquate de la tige par rapport au cotyle, et d'autre part, dans laquelle, l'orientation donnée du cotyle par rapport à la tige s'obtient en sélectionnant le col prothétique approprié en fonction du positionnement de la tige dans le fémur.

Cette tige fémorale perfectionnée pour prothèse de hanche, réalisée en deux parties distinctes, respectivement :
- une tige proprement dite, destinée à être insérée dans le fémur à renforcer, comprenant :
   - une portion proximale raccordée à un col prothétique, et présentant une embase de raccordement audit col ;
   - et une portion distale ;
- un col prothétique choisi parmi une pluralité de cols prothétiques, dont l'angle α par rapport au plan médian P de la tige varie et est compris entre plus et moins trente degrés, le col étant destiné à dépasser du fémur, dont l'extrémité reçoit une sphère, et dont la base présente une embase complémentaire de l'embase proximale de la tige,
ladite embase de raccordement de la tige audit col prothétique étant située dans un plan non perpendiculaire à l'axe longitudinal de ladite tige, et les deux embases complémentaires présentant des moyens complémentaires de solidarisation du col sur la tige.

En d'autres termes, l'invention consiste outre à ménager l'embase de raccordement col prothétique/tige dans un plan non perpendiculaire à l'axe du col prothétique, et à disposer l'embase de raccordement au niveau de la zone d'enfouissement, ce qui permet ainsi d'éviter d'entailler le grand trochanter, surtout à proposer une pluralité de cols prothétiques au praticien, cols dont l'angle par rapport au plan longitudinal de la tige est variable et permet ainsi de sélectionner ce col en fonction des nécessités, notamment d'implantation de la tige dan le fémur.

Dans une première forme d'exécution préférée, les moyens complémentaires de solidarisation sont constitués respectivement par une portée cylindrique d'emmanchement mâle, disposée sur la base du col, et par un logement cylindrique correspondant ménagé en regard dans l'embase de la tige, la portée mâle présentant un logement annulaire destiné à coopérer avec l'extrémité d'une vis d'immobilisation, ménagée à cet effet dans un logement filetée taillé dans le haut de la tige. De préférence, les deux embases complémentaires ont une forme générale en V asymétrique, à savoir une embase proprement dite, associée à une petite face inclinée, formant butée anti-rotatoire.

Dans une autre forme d'éxécution de l'invention, l'embase du col prothétique est totalement plane, et les moyens de solidarisation sont constitués par :
- un cone morse émergeant perpendiculairement de l'embase du col prothétique, destiné à coopérer avec un logement de forme et de dimension complémentaires réalisé en regard dans l'embase de la tige ;
- une vis de verrouillage, destinée à venir s'insérer dans un évidement ménagé à cet effet au niveau de l'embase dudit col, et à venir à coopérer avec un orifice taraudé de manière complémentaire ménagé dans l'embase de la tige.

Avantageusement la vis de verrouillage et le cone sont disposés dans un même plan, lui-même coplanaire avec le plan médian de la tige. En outre, cette vis et ce cone forment entre eux un angle compris entre 10 et 20°, et avantageusement 15°, de sorte qu'ils concourent au niveau de la partie proximale de la tige.

La manière dont l'invention peut être réalisée et les avantages qui en découlent ressortiront des exemples de réalisation qui suivent donnés à titre indicatif et non limitatif à l'appui des figures annexées.

La figure 1 est une représentation schématique vue de face d'une tige caractéristique de l'invention.

La figure 2 est une vue de côté de cette tige, montrée suivant l'axe F2 perpendiculaire à l'axe longitudinal P de la tige, alors que la figure 3 est une vue de dessus montrée suivant l'axe F1 longitudinal du col prothétique.

La figure 4 est une représentation détaillée de l'assemblage caractéristique de l'invention.

La figure 5 est une représentation schématique vue de côté d'une tige caractéristique de l'invention selon une autre forme de réalisation.

La figure 6 est une représentation d'un détail de la partie proximale de la tige conforme à celle représentée sur la figure 5.

La figure 7 est une représentation schématique du col prothétique conforme à la représentation de la figure 5.

La figure 8 est une autre représentation de ce col prothétique.

La figure 9 est une représentation schématique du col prothétique selon différentes inclinaisons.

La figure 10 est une représentation schématique représentant différentes inclinaisons possibles du col prothétique par rapport au plan médian de la tige.

En se référant aux figures, la tige selon l'invention comprend essentiellement deux parties, à savoir un col prothétique désigné par la référence générale (1) et une tige proprement dite désignée par la référence générale (10).

Le col prothétique (1), destiné à dépasser du fémur à renforcer, comprend dans l'ordre une tête (2), légèrement tronconique, destinée à recevoir par emmanchement une sphère non représentée sur laquelle s'appuie la cupule cotyloïdienne. Cette tête tronconique (2) est raccordée de manière connue à un col (3), puis à une base (4), dont l'embase (5) est perpendiculaire à l'axe F1 du col prothétique (1). Cette embase (5) présente en son milieu et dirigée vers l'extérieur, une portée cylindrique d'emmanchement (6), orthogonale à l'embase (5). Dans une variante,cette portée (6) peut être légèrement tronconique (cone de morse de 3 à 5°). L'extrémité (7) de cette portée cylindrique (6) est légèrement biseautée.

Selon une autre caractéristique de l'invention, cette portée cylindrique (6) présente sensiblement en son milieu une gorge annulaire (8) en forme de V.

La tige (10) comprend de manière connue une portion proximale désignée par la référence générale (11), une portion distale désignée par la référence générale (12), et une pointe (13). Cette tige (10) est destinée à être insérée dans le canal médullaire du fémur à renforcer. Selon une caractéristique de l'invention, la tige (10) et plus exactement la portion proximale (11) présente, à la limite de la zone d'enfouissement, désignée par la référence générale E, une embase (15) parallèle à l'embase (5) du col (1), donc perpendiculaire à l'axe F1, de manière à être complémentaire de cette embase (5).

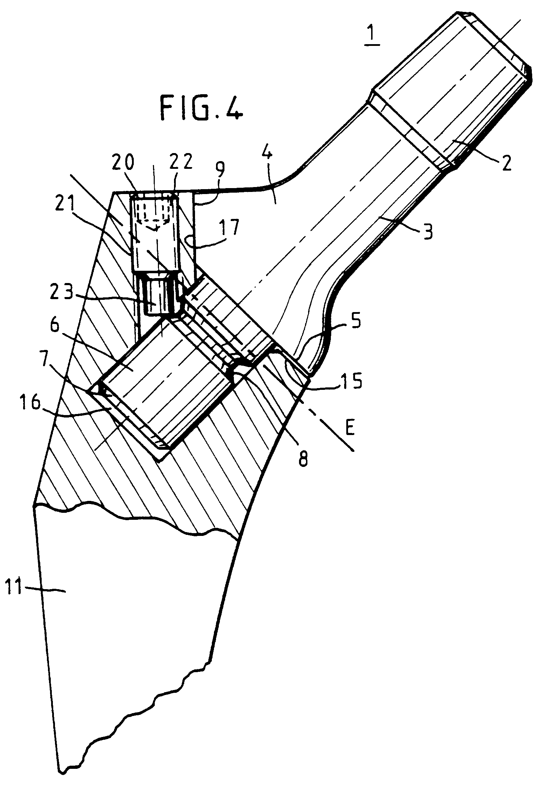

Selon une autre caractéristique de l'invention, l'embase (5) de la tige présente un logement cylindrique (16) (voir figure 4), dans lequel s'insère cote pour cote la portée de l'emmanchement (6) et disposé en regard de celle-ci. Les deux embases complémentaires (5,15) se prolongent chacune par une petite face inclinée respectivement (9) et (17), parallèles à l'axe longitudinal de la tige (10), c'est-à-dire parallèles au plan P, de manière à ce que ces deux embases (5,9; 15, 17) aient une forme générale en V asymétrique pour former butée anti-rotatoire.

La partie supérieure (20) de la portion proximale (11) présente un canal fileté (21) parallèle aux faces (9,17), donc avec le plan médian P de la tige, destiné à recevoir une vis (22) dont l'extrémité (23) forme têton pour venir s'engager dans la gorge (8), et plus exactement pour prendre appui sur une des faces de celle-ci, et par là immobiliser le col prothétique (1) dans la tige (10).

Ce canal (21) dépasse légèrement de la zone d'enfouissement (E) et est donc facilement accessible au chirurgien.

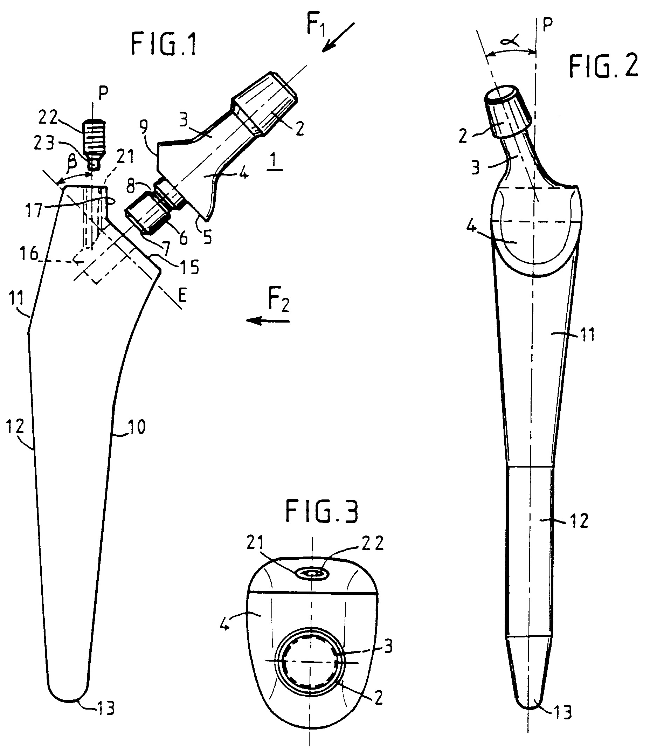

Selon une autre caractéristique essentielle de l'invention (voir figure 2), l'angle α du col prothétique (1) par rapport au plan médian P de la tige est variable d'un col par rapport à l'autre, par exemple de cinq en cinq degrés et entre plus et moins trente degrés par rapport à ce plan P. Cet angle α qui varie par construction, permet ainsi au chirurgien en fonction du positionnement du cotyle, de choisir le col prothétique (1) le plus approprié.

Le fait que les deux embases de raccordement (5,15) complémentaires soient disposées à la limite, mais juste en-dessus de la zone d'enfouissement E, permet avantageusement d'éviter d'entailler le grand trochanter. Le fait que les deux parties (1,10) soient solidarisées par un système gorge (8)/tenon (23), assure à l'ensemble une excellente tenue. Le fait que le col prothétique (1) soit incliné par rapport au plan médian P de la tige (10), permet d'insérer la tige (10) dans le canal médullaire, puis d'adapter selon le besoin et l'architecture le positionnement du col (1) par rapport au cotyle, et ce quelle que soit l'orientation de la cavité cotyloïdienne.

En outre, comme la prothèse est réalisée en deux parties distinctes, lors de la mise en place, le chirurgien n'est plus gêné par le col (1) qui est fixé ultérieurement grâce à l'assemblage vis (22)/tenon (23)/gorge (8).

Avantageusement, en pratique, comme déjà dit, l'angle α varie de cinq en cinq degrés dans une fourchette comprise entre plus et moins trente degrés par rapport au plan médian P de la tige. De même, la longueur du col prothétique (1) est comprise entre trente et cinquante millimètres, et l'angle β formé par l'embase (15) proximale avec l'axe longitudinal de la tige, est voisin de quarante huit à cinquante degrés.

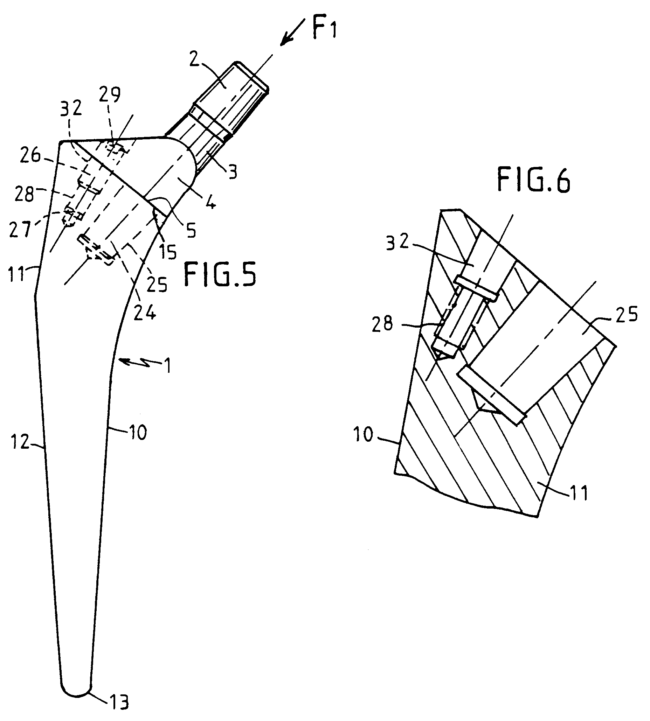

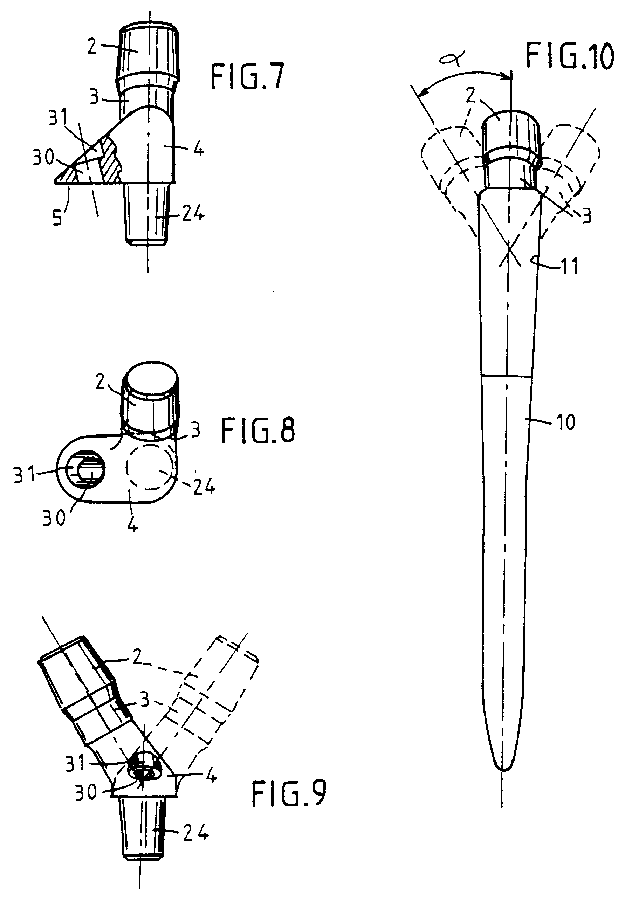

Selon une seconde forme de réalisation plus particulièrement représentée dans les figures 5 à 10, le col prothétique (1), comporte une embase (5) totalement plane et perpendiculaire à l'angle F1 du col. Ce col prothétique comporte comme moyen de solidarisation un cone morse (24) de forme légèrement conique, destiné à coopérer avec un évidemment complémentaire (25) ménagé de l'embase (15) de la partie proximale (11) de la tige, et en regard dudit cone morse lorsque le col est en place sur la tige.

Corrélativement, le col (1) comporte également un orifice (30) traversant situé dans le même plan que ledit cone morse (24), et en outre situé dans le même plan que le plan médian P de la tige (10).

Cet évidemment (30) est destiné à permettre l'insertion d'une vis de verrouillage (26), destinée à coopérer avec un évidemment correspondant (32) ménagé dans l'embase de la partie proximale (11) de la tige, et plus précisément à l'extrémité inférieure (28) taraudée, destinée à coopérer avec le filetage (27) de l'extrémité de la vis (26).

Selon une caractéristique avantageuse de l'invention, le cone morse (24) et la vis (26) sont concourants lorsque le cone est en place sur la tige, de telle sorte, à d'une part, réduire l'encombrement des évidemments correspondants au niveau de la tige, et d'autre part, à renforcer la résistance mécanique de la tige notamment en flexion. Comme on l'a représenté sur la figure 5, la tête de la tige (29) est escamoté lorsque la vis est en place. De la sorte, on évite au col prothétique de former came lors de certains mouvements du patient sur lequel est mis en place la prothèse.

Ce système de fixation s'avère particulièrement avantageux. En effet, tout d'abord la coopération du cone morse (24) avec l'évidemment complémentaire (25) ménagé dans la tige est autobloquant puisqu'il y a adhérence du cone sur cet évidemment. En outre, cette adhérence est verrouillée au moyen de la vis (26) munie du filetage (27).

Ces différents résultats se traduisent par une solidarisation plus sûre du col sur la tige, augmentant la résistance aux efforts de cisaillement et de flexion, compte tenu d'un tenon à section plus importante, la vis de verrouillage participant également à la tenue mécanique des différentes pièces.

La prothèse selon l'invention se caractérise par sa simplicité, sa facilité de mise en oeuvre, et par le fait qu'elle n'affaiblit pas l'os à renforcer. En outre, compte tenu du grand nombre de cols prothétiques adaptables sur une tige donnée, cela confère au praticien une très grande modularité, favorisant ainsi l'adaptation de la prothèse au patient. Cette multiplicité d'adaptation est en outre accentuée par le fait que la longueur de la tête (2) du col est variable. Enfin, compte tenu de la structure même de l'ensemble tige-col, la résistance mécanique des prothèses ainsi réalisées se trouve grandement accrue, notamment pour les efforts de cisaillement et de flexion.

## Revendications

1. Tige fémorale perfectionnée pour prothèse de hanche réalisée en deux parties distinctes, respectivement :
- une tige proprement dite (10), destinée à être insérée dans le fémur à renforcer, comprenant :
• une portion proximale (11) raccordée à un col prothétique (1), et présentant une embase de raccordement (15) audit col (1) ;
• et une portion distale ;
- un col prothétique choisi parmi une pluralité de cols prothétiques (1), dont l'angle α par rapport au plan médian P de la tige (10) varie et est compris entre plus et moins trente degrés, le col étant destiné à dépasser du fémur, dont l'extrémité (2) reçoit une sphère, et dont la base (4) présente une embase (5) complémentaire de l'embase proximale (15) de la tige (10) ,
ladite embase (15) de raccordement de la tige (10) audit col prothétique (1) étant située dans un plan non perpendiculaire à l'axe longitudinal de ladite tige (10), et les deux embases complémentaires (5, 15) présentant des moyens complémentaires (4, 6, 7, 8, 16, 17, 18, 24-31) de solidarisation du col (1) sur la tige (10).

2. Tige fémorale selon la revendication 1, caractérisée en ce que les moyens complémentaires de solidarisation sont constitués respectivement par une portée cylindrique (6) d'emmanchement mâle disposée sur l'embase (5) du col (1), et par un logement cylindrique complémentaire (16) ménagé en regard dans l'embase (5) de la tige (10), la portée mâle (6) présentant un logement annulaire (8), destiné à coopérer avec l'extrémité (23) d'une vis (22) d'immobilisation ménagée dans un logement fileté (21) taillé dans le haut (20) de la tige (10).

3. Tige fémorale perfectionnée selon l'une des revendications 1 et 2, caractérisée en ce que les deux embases complémentaires (5,15) ont une forme générale en V asymétrique et comprennent une grande embase (5,15) proprement dite, associée à une petite face inclinée (9,17) parallèle à l'axe longitudinal de la tige, de manière à former butée antirotatoire.

4. Tige fémorale selon l'une des revendications 1 à 3, caractérisée en ce que l'angle α du col prothétique (1) par rapport au plan médian P de la tige (10) varie de cinq en cinq degrés d'un col (1) à l'autre de la pluralité de cols prothétiques parmi lequel ledit col est choisi.

5. Tige fémorale selon la revendication 1, caractérisée en ce que l'embase (5) du col prothétique (1) est totalement plane, et en ce que les moyens de solidarisation sont constitués :
- par un cone-morse (24), emergeant perpendiculairement de l'embase (5) du col (1), destiné à coopérer avec un logement (25), de forme et dimension complémentaires, réalisé dans la partie proximale (11) de la tige (10), et débouchant au niveau de l'embase (15) de celle**-**ci en regard dudit cone (24) ;
- par une vis de verrouillage (26), destinée à venir s'insérer dans un évidement (30) ménagé à cet effet au niveau de l'embase (5) du col (1), et destinée à coopérer avec un orifice taraudé (28) de manière complémentaire, ménagé dans la partie proximale (11) de la tige (10), et débouchant au niveau de l'embase (15).

6. Tige fémorale selon la revendication 5, caractérisée en ce que la vis de verrouillage (26) et le cone-morse (24) sont situés dans le même plan, lui-même coplanaire avec le plan médian P de la tige (10).

7. Tige fémorale selon l'une des revendications 5 et 6, caractérisée en ce que la vis de verrouillage (26) et le cone-morse (24) sont concourants, l'angle qu'ils définissent étant compris entre 10 et 20 degrés.

## Claims

1. Improved femoral stem for a hip prosthesis, made up of two separate parts, namely:
- a stem proper (10) intended to be inserted into the femur to be strengthened, comprising:
• a proximal portion (11) connected to a prosthetic neck (1), and having a shoulder (15) for connection to the said neck (1);
• and a distal portion;
- a prosthetic neck chosen from among a plurality of prosthetic necks (1), of which the angle α in relation to the mid-plane P of the stem (10) varies and is between plus and minus thirty degrees, the neck being intended to protrude from the femur, and of which the end (2) receives a ball, and of which the base (4) has a shoulder (5) which complements the proximal shoulder (15) of the stem (10),
the said shoulder (15) for connecting the stem (10) to the said prosthetic neck (1) being situated in a plane not perpendicular to the longitudinal axis of the said stem (10), and the two complementary shoulders (5, 15) having complementary means (4, 6, 7, 8, 16, 17, 18, 24-31) for securing the neck (1) on the stem (10).

2. Femoral stem according to Claim 1, characterized in that the complementary securing means consist, respectively, of a cylindrical bearing surface (6), for male engagement, arranged on the shoulder (5) of the neck (1), and of a complementary cylindrical seat (16) formed opposite in the shoulder (5) of the stem (10), the male bearing surface (6) having an annular seat (8) intended to cooperate with the end (23) of a fixing screw (22) formed in a threaded seat (21) cut in the top (20) of the stem (10).

3. Improved femoral stem according to one of Claims 1 and 2, characterized in that the two complementary shoulders (5, 15) have the general shape of an asymmetrical V and comprise a large shoulder (5, 15) proper, together with a small inclined face (9, 17) parallel to the longitudinal axis of the stem, in such a way as to form an anti-rotation stop.

4. Femoral stem according to one of Claims 1 to 3, characterized in that the angle α of the prosthetic neck (1) in relation to the mid-plane P of the stem (10) varies by five degrees at a time from one neck (1) to another of the plurality of prosthetic necks from among which the said neck is chosen.

5. Femoral stem according to Claim 1, characterized in that the shoulder (5) of the prosthetic neck (1) is totally plane, and in that the securing means consist:
- of a Morse cone (24) protruding perpendicularly from the shoulder (5) of the neck (1) and intended to cooperate with a seat (25), of complementary shape and size, formed in the proximal part (11) of the stem (10), and opening out at the level of the shoulder (15) thereof opposite the said cone (24);
- of a locking screw (26) intended to be inserted in a recess (30) formed for this purpose at the level of the shoulder (5) of the neck (1) and intended to cooperate with a complementary tapped orifice (28) formed in the proximal part (11) of the stem (10) and opening out at the level of the shoulder (15).

6. Femoral stem according to Claim 5, characterized in that the locking screw (26) and the Morse cone (24) are situated in the same plane, itself coplanar with the mid-plane P of the stem (10).

7. Femoral stem according to one of Claims 5 and 6, characterized in that the locking screw (26) and the Morse cone (24) run together, the angle which they define being between 10 and 20 degrees.

## Patentansprüche

1. Verbesserter Oberschenkelknochenschaft für eine Hüftgelenksprothese, der aus zwei unterschiedlichen Teilen aufgebaut ist, und zwar:
- einem eigentlichen Schaft (10), der dazu bestimmt ist, in den zu verstärkenden Oberschenkelknochen eingesetzt zu werden, mit:
• einem proximalen Abschnitt (11), der mit einem Prothesenhals (1) verbunden ist und eine Sitzfläche (15) zur Verbindung mit dem Hals (1) aufweist;
• und einem distalen Abschnitt;
- einem Prothesenhals, der aus einer Mehrzahl von Prothesenhälsen (1) ausgewählt ist, deren Winkel α bezüglich der Mittelebene P des Schaftes (10) verschieden ist und im Bereich zwischen plus dreißig und minus dreißig Grad liegt, wobei der Hals dazu bestimmt ist, über den Oberschenkelknochen hinauszuragen, und wobei dessen äußeres Ende (2) eine Kugel aufnimmt und dessen Basis (4) eine Sitzfläche (5) aufweist, die komplementär zu der proximalen Sitzfläche (15) des Schaftes (10) ist,
wobei die Sitzfläche (15) zur Verbindung des Schaftes (10) mit dem Prothesenhals (1) in einer Ebene liegt, die nicht rechtwinklig zur Längsachse des Schaftes (10) verläuft, und wobei die beiden komplementären Sitzflächen (5, 15) komplementäre Mittel (4, 6, 7, 8, 16, 17, 18, 24-31) zur Befestigung des Halses (1) auf dem Schaft (10) aufweisen.

2. Oberschenkelknochenschaft nach Anspruch 1, dadurch gekennzeichnet, daß die komplementären Mittel zur Befestigung aus einem männlichen zylindrischen Einsetzzapfen (6), der auf der Sitzfläche (5) des Halses (1) angeordnet ist, und aus einer zylindrischen komplementären Ausnehmung (16) gebildet werden, die gegenüberliegend in der Sitzfläche (5) des Schaftes (10) vorgesehen ist, wobei der Zapfen (6) eine ringförmige Ausnehmung (8) aufweist, die dazu bestimmt ist, mit dem äußeren Ende (23) einer Feststellschraube (22) zusammenzuwirken, die in einer mit einem Gewinde versehenen Ausnehmung (21) vorgesehen ist, die in das obere Teil (20) des Schaftes (10) geschnitten ist.

3. Oberschenkelknochenschaft nach einem der Ansprüche 1 und 2, dadurch gekennzeichnet, daß die beiden komplementären Sitzflächen (5, 15) eine Grundform eines asymmetrischen V haben und eine eigentliche große Sitzfläche (5, 15) aufweisen, die mit einer kleinen geneigten Fläche (9, 17), die parallel zur Längsachse des Schaftes verläuft, in Verbindung steht, um einen drehfesten Anschlag zu bilden.

4. Oberschenkelknochenschaft nach einem der Ansprüche 1 bis 3, dadurch gekennzeichnet, daß der Winkel α des Prothesenhalses (1) bezüglich der Mittelebene P des Schaftes (10) von einem Hals (1) der Mehrzahl der Prothesenhälse, aus denen der Hals ausgewählt ist, zum anderen um jeweils 5 Grad verschieden ist.

5. Oberschenkelknochenschaft nach Anspruch 1, dadurch gekennzeichnet, daß die Sitzfläche (5) des Prothesenhalses (1) vollkommen eben ist, und daß die Mittel zur Befestigung gebildet werden aus:
- einem Morsekonus (24), der rechtwinklig aus der Sitzfläche (5) des Halses (1) hervorragt, und der dazu bestimmt ist, mit einer Ausnehmung (25) von komplementärer Form und Abmessung zusammenzuwirken, die in dem proximalen Teil (11) des Schaftes (10) ausgebildet ist und im dem Konus (24) gegenüberliegenden Bereich der Sitzfläche (15) desselben mündet;
- einer Arretierschraube (26), die dazu bestimmt ist, in eine zu diesem Zweck im Bereich der Sitzfläche (5) des Halses (1) vorgesehene Ausbohrung (30) eingesetzt zu werden, und die ferner dazu bestimmt ist, mit einer Öffnung (20) zusammenzuwirken, in die ein komplementäres Gewinde geschnitten ist, und die in dem proximalen Teil (11) des Schaftes (10) vorgesehen ist und im Bereich der Sitzfläche (15) mündet.

6. Oberschenkelknochenschaft nach Anspruch 5, dadurch gekennzeichnet, daß die Arretierschraube (26) und der Morsekonus (24) in derselben Ebene liegen, die ihrerseits mit der Mittelebene P des Schaftes (10) koplanar ist.

7. Oberschenkelknochenschaft nach einem der Ansprüche 5 und 6, dadurch gekennzeichnet, daß die Arretierschraube (26) und der Morsekonus (24) aufeinander zu verlaufend ausgebildet sind, wobei der durch sie definierte Winkel im Bereich zwischen 10 und 20 Grad liegt.
